(19) Europäisches Patentamt
European Patent Office
Office européen des brevets

(11) Veröffentlichungsnummer : **0 144 975**
**B1**

(12)

# EUROPÄISCHE PATENTSCHRIFT

(45) Veröffentlichungstag der Patentschrift : **06.05.87**

(51) Int. Cl.⁴ : **C 08 G 63/68**, C 08 G 63/76, A 61 K 7/075

(21) Anmeldenummer : **84114822.4**

(22) Anmeldetag : **06.12.84**

(54) **Quaternäre oxalkylierte Polyester, Verfahren zu deren Herstellung und deren Verwendung.**

(30) Priorität : **14.12.83 DE 3345156**

(43) Veröffentlichungstag der Anmeldung : **19.06.85 Patentblatt 85/25**

(45) Bekanntmachung des Hinweises auf die Patenterteilung : **06.05.87 Patentblatt 87/19**

(84) Benannte Vertragsstaaten : **BE CH DE FR GB IT LI NL SE**

(56) Entgegenhaltungen :
**DE-A- 3 032 216**
**FR-A- 2 112 454**
**FR-A- 2 331 324**
**GB-A- 2 006 237**
**US-A- 3 816 378**

(73) Patentinhaber : **HOECHST AKTIENGESELLSCHAFT**
**Postfach 80 03 20**
**D-6230 Frankfurt am Main 80 (DE)**

(72) Erfinder : **Hofinger, Manfred, Dr.**
**Hoher Göll Weg 7**
**D-8269 Burgkirchen (DE)**
Erfinder : **Reng, Alwin**
**Im Schulzehnten 22**
**D-6233 Kelkheim (Taunus) (DE)**
Erfinder : **Quack, Jochen Meinhard, Dr.**
**Wilhelm-Reuter-Strasse**
**D-6239 Eppstein/Taunus (DE)**

Anmerkung : Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

EP 0 144 975 B1

Jouve, 18, rue St-Denis, 75001 Paris, France

**Beschreibung**

Das menschliche Haar kann durch äußere Einflüsse, insbesondere durch physikalische, chemische und biologische Einflüsse angegriffen und, gegebenenfalls irreparabel, geschädigt werden. Zur Prophylaxe, zur Minderung solcher Einflüsse und zur kosmetischen Nachbehandlung des gewaschenen Haares sind neben anderen Mitteln verbreitet quaternäre Ammoniumverbindungen in Haarnachspülmitteln eingesetzt worden. Mit Hilfe solcher substantiver quaternärer Ammoniumverbindungen lassen sich die gewünschten Konditioniereffekte auf dem Haar erzielen, wobei darunter vor allem eine erleichterte Naß- und Trockenkämmbarkeit des Haares, ein weicher Griff und Volumen und Fülle des gewaschenen Haares zu verstehen ist. Beispiele für solche quaternären Ammoniumverbindungen sind Alkyltrimethylammoniumhalogenide, Dialkyldimethylammoniumhalogenide, Alkyldimethylbenzylammoniumhalogenide (wobei unter Alkyl hier jeweils ein langkettiger Fettalkylrest zu verstehen ist), oxalkylierte quaternäre Ammoniumverbindungen sowie heterocyclische quaternäre Ammoniumverbindungen, wie beispielsweise Imidazolinium- oder Morpholiniumverbindungen.

Um den bei der Anwendung von Haarnachspülmitteln erforderlichen Arbeitsgang einzusparen, wurde auch schon versucht, quaternäre Ammoniumsalze in Haarwaschmittel einzuarbeiten. Zusammen mit den dort üblicherweise anwesenden anionischen Tensiden bilden sich dabei jedoch sogenannte Elektroneutralsalze, die die erwünschten Konditioniereffekte auf dem Haar nur noch in sehr vermindertem Maße bewirken können.

Eine Verbesserung bringt die Verwendung von polymeren quaternären Ammoniumsalzen in Haarwaschmitteln. Dies sind zum Beispiel Cellulosederivate, welche quaternäre Ammoniumgruppen tragen und beispielsweise aus der US-PS 3 472 840 bekannt sind ; ferner insbesondere Polyvinylpyrrolidone beziehungsweise Copolymere des Vinylpyrrolidons mit Acrylaten oder Methacrylaten (vgl. beispielsweise DE-OS 2 103 898), welche quaternäre Ammoniumgruppen tragen ; oder auch Copolymere von Dialkenyldialkylammoniumsalzen und Acrylamid oder Methacrylamid, wie sie in der DE-OS 3 029 306 beschrieben sind.

Die Anwendung solcher hochmolekularer quaternärer Ammoniumverbindungen ergibt nach der ersten Haarwäsche zufriedenstellende Konditioniereffekte, die jedoch im Vergleich zur alleinigen Anwendung der eingangs erwähnten niedermolekularen quaternären Ammoniumsalze erheblich schwächer ausgeprägt sind. Weitere Nachteile, die aus dem Einsatz solcher polymerer quaternärer Ammoniumverbindungen resultieren, sind ein starker Akkumuliereffekt bei wiederholter Anwendung solcher Haarwaschmittel und die nicht völlige Klarlöslichkeit in den üblicherweise in Haarwaschmitteln anwesenden anionischen Tensiden.

Aus der DE-OS 3 032 216 sind Umsetzungsprodukte von oxalkylierten tertiären Aminen mit Dicarbonsäuren und deren Salze mit Mineralsäuren oder kurzkettigen Carbonsäuren bekannt, wobei die so hergestellten aminhaltigen Polyester und deren Salze einen Polymerisationsgrad von 2 bis 50 haben und sowohl in Haarwasch- als auch in Haarnachspülmitteln Einsatz finden sollen. Die polymeren Amine und insbesondere ihre Salze weisen für einen solchen Einsatz jedoch folgende nachteilige Eigenschaften auf : Sie sind auf dem Haar schlecht verteilbar ; das überschüssige Produkt ist schwer wieder ausspülbar und ergibt einen unangenehm glitschigen Griff des so behandelten nassen Haares. Außerdem weisen Amine und Aminsalze eine häufig nicht zufriedenstellende dermatologische Verträglichkeit auf. Es besteht daher ein Bedürfnis nach Substanzen, die die vorgenannten Nachteile nicht aufweisen.

Zur Befriedigung dieses Bedürfnisses werden gemäß der vorliegenden Erfindung quaternäre oxalkylierte Polyester der allgemeinen Formel

$$HO\left[\overset{O}{\overset{\|}{C}}-R^1-\overset{O}{\overset{\|}{C}}-(O-CHR^2-CH_2)_a-\underset{R^3}{\underset{|}{\overset{R^4}{\overset{|}{N^+}}}}-(CH_2-CHR^2O)_b\right]_n H \quad n\cdot A^- \qquad (I)$$

worin

$R^1$ ein Alkylenrest der Formel $-(CH_2)_m-$, in der m eine ganze Zahl von 1 bis 8 bedeutet und wobei dieser Alkylenrest gegebenenfalls 1 bis 2 OH-Gruppen tragen kann, ein Vinylen- oder ein p-Phenylenrest ist,

$R^2$ H oder $CH_3$ ist,

$R^3$ einen Alkylrest oder Alkenylrest mit 8 bis 23 C-Atomen bedeutet,

$R^4$ einen Rest der Formel $-(CH_2CHR^2O)_c-H$, in der c eine ganze oder gebrochene Zahl von 1 bis 3 ist und $R^2$ die obige Bedeutung hat, bedeutet,

$A^-$ das Anion einer Carbonsäure mit 2 bis 6 C-Atomen, einer Hydroxycarbonsäure mit 2 bis 6 C-Atomen und 1 bis 3 OH-Gruppen, das Chlorid- oder das Hydrogenphosphatanion bedeutet,

a und b, gleich oder verschieden, eine ganze oder gebrochene Zahl von 1 bis 10 ist, und

n eine ganze oder gebrochene Zahl ist, die Werte von 5 bis 60 annehmen kann,

zur Verfügung gestellt.

Vorzugsweise ist in der Formel I

$R^1$ ein Alkylenrest der Formel $-(CH_2)_m-$, in der m Werte von 4 bis 8 annehmen kann,

$R^2 = H$,

$R^3$ ein Alkylrest mit 16 bis 23 C-Atomen,

$R^4$ ein Rest der Formel $-(CH_2CH_2O)_c-H$, in der c eine ganze oder gebrochene zahl von 1 bis 3 bedeutet,

a und b, gleich oder verschieden, eine ganze oder gebrochene Zahl von 2 bis 4

n eine ganze oder gebrochene Zahl von 5 bis 20, und

$A^-$ das Anion der Milchsäure, der Weinsäure oder der Phosphorsäure.

Die Verbindungen der Formel I werden erhalten, indem man ein oxalkyliertes primäres Fettamin der Formel

$$\begin{array}{l} \quad\;\; (CH_2CHR^2O)_a-H \\ \quad\;\; | \\ R^3-N \\ \quad\;\; | \\ \quad\;\; (CH_2CHR^2O)_b-H \end{array} \qquad (II)$$

in der $R^3$, $R^2$, a und b die in Formel I angegebenen Bedeutungen haben, mit einer Dicarbonsäure der Formel

$$HOOC-R^1-COOH \qquad (III),$$

worin $R^1$ die in Formel I angegebene Bedeutung hat, unter Polykondensation verestert, wobei das Verhältnis von oxalkyliertem primärem Fettamin zu Dicarbonsäure 0,8 : 1 bis 1 : 0,8 beträgt, und das erhaltene Reaktionsprodukt mit Ethylenoxid, Propylenoxid oder Gemischen dieser beiden Alkylenoxide, jeweils zusammen mit einer Carbonsäure, mit Phosphorsäure oder Salzsäure umsetzt.

Die Ausgangsverbindungen der Formel II werden erhalten nach bekannten Verfahren durch Oxalkylierung von primären Fettaminen. Eine Übersicht über Methoden zur Herstellung dieser wohlbekannten Verbindungsklasse wird gegeben in Schönfeldt, « Surface Active Ethyleneoxide Adducts », Wissenschaftliche Verlagsgesellschaft, Stuttgart, 1976, Seiten 70 bis 73.

Bevorzugte Amine, die zu Ausgangsverbindungen der Formel II oxalkyliert werden können, sind die technisch verfügbaren Produkte Stearylamin oder Behenylamin. Es ist aber gemäß der Erfindung ebensogut möglich, andere Monoamine mit mehr oder weniger breiter Alkylkettenverteilung oder auch Amine mit einheitlicher Kette zu verwenden. Es können auch Fettamine einzeln oder im Gemisch eingesetzt werden, deren Ketten eine oder mehrere Doppelbindungen enthalten, wie die Reste der Öl-, Elaidin-, Linol- oder Linolensäure.

Zur Veresterung geeignete Dicarbonsäuren sind aliphatische Dicarbonsäuren mit $C_1$- bis $C_8$-Alkylengruppen, wie Malonsäure, Bernsteinsäure, Glutarsäure, Adipinsäure, Sebacinsäure, aliphatische Dicarbonsäuren, die durch 1 bis 2 OH-Gruppen substituiert sind, wie Äpfelsäure, Tartronsäure, Weinsäure sowie ferner auch die Terephthalsäure, die Fumarsäure oder die Maleinsäure. Besonders geeignet sind die aliphatischen Dicarbonsäuren mit 4 bis 8 C-Atomen im Alkylenrest, also die homologe Reihe von der Adipin- bis zur Sebacinsäure. Zur Veresterung geeignet sind auch Derivate solcher Dicarbonsäuren, insbesondere deren Ester und Säurehalogenide.

Die unter Polykondensation verlaufende Veresterung der Verbindungen der Formel II erfolgt nach bekannten Methoden mit einer Dicarbonsäure der Formel III in höhersiedenden inerten Lösungsmitteln, wie Toluol oder Xylol, oder bevorzugt ohne Lösungsmittel in der Schmelze und unter Abdeckung mit einem Schutzgas. Bei Veresterung in einem Lösungsmittel wählt man als Reaktionstemperatur zweckmäßigerweise die Rückflußtemperatur des Reaktionsgemisches und entfernt das gebildete Reaktionswasser azeotrop. Bei Veresterung in Substanz wird das Reaktionswasser direkt aus der Reaktionsmischung abdestilliert. Die Reaktiontemperaturen liegen hier bei 140 bis 220 °C bevorzugt bei 150 bis 180 °C. Zur Beschleunigung der Reaktion verwendet man einen sauren Katalysator, wie zum Beispiel p-Toluolsulfonsäure oder hypophosphorige Säure. Die Vollständigkeit der Reaktion wird über die Bestimmung der Amin- und Säurezahl kontrolliert. Das Molverhältnis von Dicarbonsäure zu Verbindungen der Formel II wird in einem bestimmten Verhältnis variiert. Diese Variation und der Umsetzungsgrad der Polykondensation haben, wie bekannt, folgenden Einfluß auf den Polykondensationsgrad $\bar{P}_n$

$$\bar{P}_n = (1 + q)/(1 + q - 2\,pq)$$

worin p den Umsetzungsgrad der Polykondensation, q das stöchiometrische Verhältnis von Dicarbonsäure zu Verbindung der Formel II bedeutet.

Variiert wird im Verhältnis oxalkyliertes Fettamin : Dicarbonsäure im Bereich 0,8 : 1 bis 1 : 0,8. Bevorzugt wendet man 0,83 bis 0,92 mol der Dicarbonsäure und 1 mol des oxalkylierten Amins an und führt den Umsatz bis nahe 100 %. Die analytische Bestimmung des mittleren Polykondensationsgrades

und damit der mittleren Molmasse wird durch HPLC (= high performance liquid chromatography) oder HPSEC (= high performance size exclusion chromatography) vorgenommen. Die Quaternisierungsreaktion wird mit Ethylen- oder Propylenoxid oder deren Gemischen, vorzugsweise mit Ethylenoxid, bei einer Temperatur von 80 bis 95 °C in einem geeigneten Rührautoklaven durchgeführt, wobei ein maximaler Reaktionsdruck von 3 bar zweckmäßigerweise nicht überschritten werden sollte. Dabei wird zur Salzbildung mit einer äquivalenten Menge (entsprechend der Zahl der N-Atome) einer derjenigen Carbonsäuren oder Mineralsäuren umgesetzt, die den oben definierten Anionen $A^-$ zugrundeliegen. Solche Carbonsäuren sind beispielsweise Essigsäure, Propionsäure, die gegebenenfalls auch 1 bis 3 Hydroxygruppen tragen können, wie Glykolsäure und Milchsäure. Die Carbonsäure kann auch eine — gegebenenfalls OH-substituierte — Di- oder Tricarbonsäure sein, wie Bernsteinsäure, Malon-, Malein-, Fumar-, Äpfel-, Wein- oder Citronensäure. Schließlich kommen als Mineralsäuren in Betracht Orthophosphorsäure oder Salzsäure. Bevorzugt sind Milchsäure, Weinsäure und Orthophosphorsäure. Die Quaternisierungsgrade werden durch Zweiphasentitration des quaternären Produktes der Formel I mit Natriumdodecylsulfat bei pH 1 bis 2 beziehungsweise pH 10 ermittelt.

Die folgenden Beispiele erläutern die Herstellung der erfindungsgemäßen Verbindungen :

Beispiel 1

a) Herstellung eines Polykondensationsproduktes aus Adipinsäure und einem Stearylamin, das mit 10 mol Ethylenoxid kondensiert ist :

In einem 1-l-Rührgefäß, versehen mit Wasserabscheider, Gaseinleitung und Heizung, werden 564,3 g (0,81 mol) Stearylamin + 10 mol Ethylenoxid, 98,6 g (0,67 mol) Adipinsäure und 1,7 g 50 gew.-%ige hypophosphorige Säure vorgelegt, unter Stickstoff-Atmosphäre auf 180 °C gebracht und unter kontinuierlicher Wasserabscheidung die Veresterungsreaktion bei dieser Temperatur fortgesetzt. Nach 15 stündiger Reaktionszeit wird nach Bestimmung der Säure- und Aminzahl ein praktisch vollständiger Kondensationsumsatz ermittelt.

b) Quaternisierung des erhaltenen Polykondensats mit Carbonsäure und Alkylenoxid.

377 g (0,45 mol, berechnet auf die wiederkehrende Einheit) des gemäß Beispiel 1a) erhaltenen Polykondensats wird in Gegenwart von 32,4 g (1,80 mol) Wasser und 28 g Ethanol mit 55,8 g (0,45 mol) 70 %iger Milchsäure neutralisiert und mit 99,1 g (2,25 mol) Ethylenoxid quaternisiert. Die Reaktion ist bei einer Temperatur von 85 °C und einem maximalen Druck von 3,0 bar in 7 Stunden beendet. Es resultiert eine bei Raumtemperatur klare Flüssigkeit. Der Quaternisierungsgrad des quaternären Polykondensationsprodukts wird aus dem Verhältnis der sauren beziehungsweise alkalischen Zweiphasentitration mit Natriumdodecylsulfat bestimmt und beträgt 94 %. Unter den in Beispiel 1 beschriebenen Reaktionsbedingungen lassen sich die in folgender Tabelle 1, Beispiele 2 bis 16, angegebenen Reaktionen zu Verbindungen der Formel I mit den aufgeführten Quaternisierungsgraden durchführen :

(Siehe Tabelle 1 Seite 5 ff.)

Die erfindungsgemäßen quaternären oxalkylierten Polyester der Formel I sind insbesondere in der Haarkosmetik universell einsetzbar. Während sich die oben erwähnten quaternären Ammoniumsalze wegen ihres Wirkungsabfalls in Gegenwart anionischer Tenside fast ausschließlich nur für Haarnachspülmittel eignen und die erwähnten Polymeren, die quaternäre Ammoniumgruppen enthalten, u. a. aus Gründen einer nicht ausreichenden Verdickung mit üblichen Verdickungsmitteln nicht für Haarnachspülmittel, sondern nur für Haarwaschmittel formulieren lassen, können die erfindungsgemäßen Verbindungen der Formel I für die verschiedensten Zwecke in der Haarkosmetik Anwendung finden : Sie eignen sich sowohl für Haarwaschmittel als auch für Haarnachspülmittel, für Haarverformungsmittel, Haarfestiger und Haarfärbemittel, für Haarregenerierungsmittel und andere haarkosmetische Zubereitungen.

Die erfindungsgemäßen quaternären oxalkylierten Polyester der Formel I können aber auch auf anderen Gebieten der Kosmetik Verwendung finden, so zum Beispiel in Hautpflegemitteln, Lotions oder in den verschiedenartigsten Hautreinigungsmitteln, insbesondere aber in flüssigen Seifen, Stückseifen oder Duschbädern, wo sie ein angenehmes Hautgefühl nach der Anwendung vermitteln.

Für die genannten Anwendungszwecke können den erfindungsgemäßen Verbindungen der Formel I anionische, nichtionische oder auch kationische oder zwitter-ionische Tenside beigegeben werden. Als nicht-ionische Tenside, die vorzugsweise in Haarnachbehandlungsmitteln Verwendung finden, kommen dafür als Stoffgruppen insbesondere in Frage oxalkylierte Fettalkohole oder Alkylphenole, vorzugsweise oxalkyliert mit Ethylenoxid oder Gemischen von Ethylen- oder Propylenoxid ; Polyglykolester von

Tabelle 1

| Bei-spiel | Säure-komponente | Amin-komponente (Molverhältnis) | Polykondensa-tionsumsatz nach SZ[4] (%) | Anion der quaternären Verbindung | Quaternisie-rungsgrad (%) |
|---|---|---|---|---|---|
| 2 | Adipinsäure 1 | Stearylamin + 2 EO[2] 1 | 97 | Lactat | 73 |
| 3 | Adipinsäure 0,833 | Stearylamin + 3,5 EO 1 | 99,5 | Lactat | 85 |
| 4 | Adipinsäure 0,833 | Stearylamin + 5 EO 1 | 99 | Lactat | 93 |
| 5 | Adipinsäure 0,92 | Stearylamin + 10 EO 1 | 99 | Lactat | 84 |
| 6 | Adipinsäure 1 | Stearylamin + 10 EO 1 | 98 | Lactat | 84 |
| 7 | Adipinsäure 0,833 | Talgfettamin + 10 EO 1 | 98,5 | Lactat | 83 |
| 8 | Adipinsäure 0,9 | Cocosamin + 5 EO 1 | 99 | Lactat | 82 |
| 9 | Adipinsäure 0,9 | Cocosamin + 20 EO 1 | 97,5 | Lactat | 81 |
| 10 | Adipinsäure 0,833 | Stearylamin + 3 EO 1 | >99 | Hydrogen-phosphat | 92 |
| 11 | Adipinsäure 0,833 | Stearylamin + 2 EO + 2PO[3] 1 | 99 | Lactat | 83 |
| 12 | Adipinsäure 0,833 | Stearylamin + 3 EO 1 | >99 | Lactat | 77[1] |

**Tabelle 1 (Fortsetzung)**

| Bei-spiel | Säure-komponente | Amin-komponente | Polykondensationsumsatz nach SZ[4]) (%) | Anion der quaternären Verbindung | Quaternisierungsgrad (%) |
|---|---|---|---|---|---|
| | Molverhältnis | | | | |
| 13 | Adipinsäure 0,833 | Stearylamin + 3 EO 1 | >99 | Chlorid | 98 |
| 14 | Adipinsäure 0,92 | Stearylamin + 3 EO 1 | | Lactat | 83 |
| 15 | Adipinsäure 1 | Stearylamin + 3 EO 0,833 | 97 | Lactat | 80 |
| 16 | Adipinsäure 0,833 | Stearylamin + 3 EO 1 | 98,8 | Lactat | 85 |

[1]) Quaternisierungsreaktion mit 80 Mol-% Milchsäure
[2]) EO = Ethylenoxid
[3]) PO = Propylenoxid
[4]) SZ = Säurezahl

Fettsäuren oder Fettsäureamiden ; Ethylenoxid-Propylenoxid-Blockpolymerisate ; Glycerinester und Polyglycerinester ; Sorbit- und Sorbitanester ; Polyglykolester des Glycerins ; oxethylierte Lanolinderivate ; Alkanolamide und Saccharoseester.

Als anionische Tenside, wie sie insbesondere für die Formulierung von Haarwaschmitteln und auch von anderen Kosmetika in Betracht kommen, seien genannt Alkylsulfate ; Alkylpolyethersulfate ; Alkylarylpolyethersulfate ; Alkylsulfonate ; Alkylarylsulfonate ; α-Olefinsulfonate ; Sulfobernsteinsäurederivate ; Alkylglycidylethersulfate und -sulfonate ; Acylalkylolamidsulfate und -sulfonate ; Acylamidopolyglykolethersulfate und -sulfonate ; Fettsäuretauride ; Fettsäureisethionate ; Eiweißkondensate ; Fettsäureseifen oder Fettsäuresarkoside.

Schließlich können auch zwitter-ionische und kationische Tenside beigegeben werden, wie beispielsweise Alkyl- und Alkylamidobetaine ; Aminoxide ; Sulfobetaine und Alkylsulfatobetaine ; Imidazoliniumverbindungen ; quaternäre Ammoniumverbindungen, insbesondere solche von Fettaminen.

Ferner können für haarkosmetische Zubereitungen zu den erfindungsgemäßen Verbindungen der Formel I auch noch übliche Zusatzstoffe gegeben werden, wie beispielsweise Konsistenzgeber, Schaumstabilisatoren, Parfümöle, Konservierungsmittel, Antischuppenmittel, Rückfettungsmittel, Färbemittel und Sequestrierungsmittel.

Die folgenden Rezepturen sollen die Möglichkeiten für die Formulierung von Kosmetika erläutern, ohne die Erfindung auf diese Beispiele zu beschränken (alle im folgenden gegebenen Prozentangaben sind Gewichtsprozent) :

a) Klares, mittelviskoses Haarshampoo

15,0 % Lauryldiglykolethersulfat-Natriumsalz
2,0 % Cocosfettsäurediethanolamid
1,5 % Verbindung aus Beispiel 3
0,3 % Parfümöl
2,0 % Natriumchlorid
ad 100,0 % Wasser

b) Cremeförmiges Haarshampoo

20,0 % Cocosfettsäuremethyltaurid-Natriumsalz
5,0 % Stearinsäuremethyltaurid-Natriumsalz
2,0 % Cocosfettsäuresarkosid-Natriumsalz
0,4 % Parfümöl
2,2 % Verbindung aus Beispiel 2
ad 100,0 % Wasser, Konservierungsmittel, Farbstoff

c) Flüssiges Handwaschmittel

10,0 % Lauryltriglykolethersulfat-Natriumsalz
3,0 % Acylaminopolyglykolethersulfat-Triethanolaminsalz
3,0 % Cocosdimethylaminoxid
3,0 % Cocosfettsäureisethionat-Natriumsalz
1,0 % Triethylenglykoldistearat
0,4 % Verbindung aus Beispiel 2
1,4 % Natriumchlorid
ad 100,0 % Wasser, Konservierungsmittel, Farbstoff

d) Haarnachspül-Konzentrat

6,6 % Verbindung aus Beispiel 2
3,0 % Cetylalkohol
1,5 % Diglycerinisostearat
ad 100,0 % Wasser, Farbstoff, Parfümöl

e) Klares Haarnachspülmittel

4,5 % Verbindung aus Beispiel 3
2,0 % Hydroxyethylcellulose
ad 100,0 % Wasser, Farbstoff, Parfümöl

f) Klares Haarnachspülmittel-Konzentrat

4,5 % Verbindung aus Beispiel 3

4,0 % quaternisiertes Etheramin der Formel

$$\{[RO(CH_2CH_2O)_5]_2N(CH_3)_2\}^-Cl^-$$

2,0 % Hydroxyethylcellulose
ad 100,0 % Wasser, Farbstoff, Parfümöl

g) Klares Haarnachspülmittel

6,0 % Verbindung aus Beispiel 3
2,0 % Cetyltrimethylammoniumchlorid
2,0 % Hydroxyethylcellulose
ad 100,0 % Wasser, Parfümöl, Farbstoff

h) Frisiercreme

0,06 % Verbindung aus Beispiel 1
8,0 % Diglycerintetraglykolstearat
2,0 % Glycerinmonostearat
15,0 % Paraffinöl (hochviskos)
5,0 % Isopropylmyristat
3,0 % Glycerin
ad 100,0 % Wasser, Parfümöl, Farbstoff, Konservierungsmittel

i) Flüssiger Haarfestiger

0,2 % Verbindung aus Beispiel 2
3,0 % Vinylpyrrolidon/Vinylacetat-Mischpolymerisat Verhältnis 60 : 40
45,0 % Isopropylalkohol
0,2 % Polyethylenglykol 400
ad 100,0 % Wasser, Parfümöl

j) Mittelviskoses Duschbad

13,0 % Lauryldiglykolethersulfat-Natriumsalz
5,0 % Acylaminopolyglykolethersulfat-Triethanolaminsalz
2,0 % Ethylenglykoldistearat
2,2 % Verbindung aus Beispiel 2
0,3 % Parfümöl
3,0 % Natriumchlorid
ad 100,0 % Wasser, Konservierungsmittel, Farbstoff

k) Öl-in-Wasser-Emulsion

0,05 % Verbindung aus Beispiel 2
6,0 % Diglycerintetraglykolstearat
10,0 % Paraffinöl, hochviskos
10,0 % Isopropylpalmitat
ad 100,0 % Wasser, Konservierungsmittel, Parfümöl.

Das anwendungstechnische Verhalten der erfindungsgemäßen quaternären oxalkylierten Polyester der Formel I wurde in vivo am Lebendhaar untersucht.

Die Vorbehandlung der Haare erfolgte in Annäherung zur Praxis durch Waschen mit einer 1 %igen Lauryldiglykol-ethersulfat-Natriumsalz-Lösung in Wasser. Anschließend wurde jeweils mit Wasser von + 35 °C ausgespült.

Die Durchführung des in vivo-Tests erfolgte nach dem sogenannten Halbkopf-Test, das heißt das Haupthaar wurde in der Mitte gescheitelt und jeweils 10 ml einer 2 %igen wäßrigen Lösung beziehungsweise Dispersion der erfindungsgemäßen Verbindung wurden mit einer Pipette auf das feuchte Kopfhaar verteilt. Nach 5 minütiger Einwirkungszeit wurde mit 2 l Leitungswasser von + 35 °C die überschüssige Menge ausgespült.

Es wurde sodann sowohl das antistatische Verhalten als auch die Kämmbarkeit mit einem Standard-Kamm geprüft.

Die Beurteilung erfolgte nach folgendem Schema :

1 = sehr gut

2 = gut
3 = mäßig
4 = schlecht
5 = sehr schlecht.

Die dabei erhaltenen Ergebnisse zeigt Tabelle 2. Wie ersichtlich, weisen die erfindungsgemäßen Verbindungen sowohl eine bessere Naßkämmbarkeit als auch eine verbesserte Trockenkämmbarkeit und höheren Glanz, verglichen mit der als Vergleichssubstanz gewählten Verbindung Cetyltrimethylammoniumchlorid, auf.

Die erfindungsgemäßen Verbindungen sind schaumarm. Bei dem Einsatz der erfindungsgemäßen Verbindungen in Haarshampoos wurde auch nach der praxisüblichen kontinuierlichen Anwendung kein sogenannter akkumulierender Effekt gefunden, der normalerweise negative Auswirkungen auf Aussehen, Griff, Fülle und Glanz der Haare besitzt. Auch ist die antistatische Wirkung besser als bei den Vergleichsprodukten.

Tabelle 2

| Produkt | Trockenkämmbarkeit | Naßkämmbarkeit | Glanz der Haare |
|---|---|---|---|
| Cetyltrimethylammoniumchlorid | 3 | 3 | 3 |
| Beispiel 1 | 2 | 3 | 2 |
| Beispiel 2 | 1 | 1 | 1 |
| Beispiel 3 | 1 - 2 | 1 - 2 | 1 - 2 |
| Beispiel 4 | 2 | 2 | 2 |
| Hydroxyethylcellulose, umgesetzt mit trimethylammoniumsubstituiertem Epoxid gemäß US-PS 3 472 840 | 3 | 4 | 3 |

**Patentansprüche**

1. Quaternäre oxalkylierte Polyester der allgemeinen Formel

$$HO\left[\overset{O}{\overset{\|}{C}}-R^1-\overset{O}{\overset{\|}{C}}-(O-CHR^2-CH_2)_a-\underset{R^3}{\overset{R^4}{N^+}}-(CH_2-CHR^2O)_b\right]_n H \quad n\cdot A^- \qquad (I)$$

worin

$R^1$ ein Alkylenrest der Formel $-(CH_2)_m-$, in der m eine ganze Zahl von 1 bis 8 bedeutet und wobei dieser Alkylenrest gegebenenfalls 1 bis 2 OH-Gruppen tragen kann, ein Vinylen- oder p-Phenylenrest ist,

$R^2$ H oder $CH_3$ ist,

$R^3$ einen Alkylrest oder Alkenylrest mit 8 bis 23 C-Atomen bedeutet,

$R^4$ einen Rest der Formel $-(CH_2CHR^2O)_c-H$, in der c eine ganze oder gebrochene Zahl von 1 bis 3 ist und $R^2$ die obige Bedeutung hat, bedeutet,

$A^-$ das Anion einer Carbonsäure mit 2 bis 6 C-Atomen, einer Hydroxycarbonsäure mit 2 bis 6 C-Atomen und 1 bis 3 OH-gruppen, das Chlorid- oder das Hydrogenphosphatanion bedeutet,

a und b, gleich oder verschieden, eine ganze oder gebrochene Zahl von 1 bis 10 ist, und

n eine ganze oder gebrochene Zahl ist, die Werte von 5 bis 60 annehmen kann.

2. Quaternäre oxalkylierte Polyester gemäß Anspruch 1, dadurch gekennzeichnet, daß

$R^1$ ein Alkylenrest der Formel $-(CH_2)_m-$, in der m Werte von 4 bis 8 annehmen kann,

$R^2 = H$,

$R^3$ ein Alkylrest mit 16 bis 23 C-Atomen,

$R^4$ ein Rest der Formel $-(CH_2CH_2O)_c-H$, in der c eine ganze oder gebrochene Zahl von 1 bis 3 bedeutet,

a und b, gleich oder verschieden, eine ganze oder gebrochene Zahl von 2 bis 4,

n eine ganze oder gebrochene Zahl von 5 bis 20, und

$A^-$ das Anion der Milchsäure, der Weinsäure oder der Phosphorsäure ist.

3. Verfahren zur Herstellung von quaternären oxalkylierten Polyestern der Formel I gemäß Anspruch 1, bei dem ein oxalkyliertes primäres Fettamin der Formel

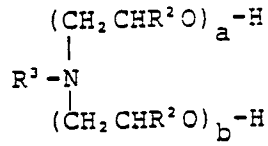

(II)

in der $R^3$, $R^2$, a und b die in Formel I angegebenen Bedeutungen haben, mit einer Dicarbonsäure der Formel

$$HOOC-R^1-COOH \quad (III),$$

worin $R^1$ die in Formel I angegebene Bedeutung hat, unter Polykondensation verestert wird, wobei das Verhältnis von oxalkyliertem primärem Fettamin zu Dicarbonsäure 0,8 : 1 bis 1 : 0,8 beträgt, dadurch gekennzeichnet, daß das erhaltene Reaktionsprodukt mit Ethylenoxid, Propylenoxid oder Gemische dieser beiden Alkylenoxide, jeweils zusammen mit einer Carbonsäure mit 2 bis 6 C-Atomen, einer Hydroxycarbonsäure mit 2 bis 6 C-Atomen und 1 bis 3 OH-Gruppen, mit Phosphorsäure oder mit Salzsäure umgesetzt wird.

4. Verwendung der quaternären oxalkylierten Polyester gemäß einem oder mehreren der Ansprüche 1 und 2 als konditionierender Wirkstoff in haarkosmetischen Zubereitungen.

**Claims**

1. A quaternary oxalkylated polyester of the formula

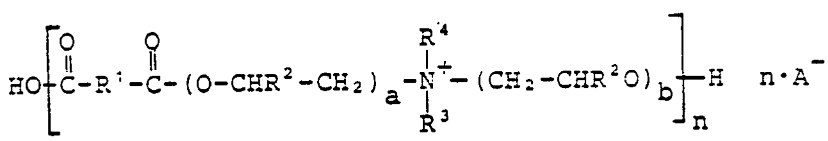

(I)

in which

$R^1$ is an alkylene radical of the formula $-(CH_2)_m-$ in which m denotes an integer from 1 to 8, this alkylene radical having, if appropriate, 1 to 2 OH groups, or is a vinylene radical or a p-phenylene radical,

$R^2$ is H or $CH_3$,

$R^3$ denotes an alkyl radical or alkenyl radical having 8 to 23 carbon atoms,

$R^4$ denotes a radical of the formula $-(CH_2CHR^2O)_c-H$ in which c is a whole or fractional number from 1 to 3 and $R^2$ has the above meaning,

$A^-$ denotes the anion of a carboxylic acid having 2 to 6 carbon atoms, of a hydroxycarboxylic acid having 2 to 6 carbon atoms and 1 to 3 OH groups, or denotes the chloride or hydrogen phosphate anion,

a and b are identical or different and are a whole or fractional number from 1 to 10, and

n is a whole or fractional number which can assume values from 5 to 60.

2. A quaternary oxalkylated polyester as claimed in claim 1, wherein

$R^1$ is an alkylene radical of the formula $-(CH_2)_m-$ in which m can assume values from 4 to 8,

$R^2$ is H,

$R^3$ is an alkyl radical having 16 to 23 carbon atoms,

$R^4$ is a radical of the formula $-(CH_2CH_2O)_c-H$ in which c denotes a whole or fractional number from 1 to 3,

a and b are identical or different and are a whole or fractional number from 2 to 4

n is a whole or fractional number from 5 to 20 and $A^-$ is the anion of lactic acid, tartaric acid or phosphoric acid.

3. A process for the preparation of quaternary oxalkylated polyesters of the formula I as claimed in claim 1, in which an oxalkylated primary fatty amine of the formula

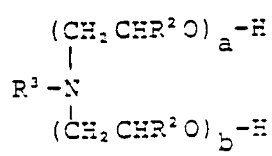

(II)

in which $R^3$, $R^2$, a and b have the meanings indicated in formula I, is esterified, with polycondensation, with a dicarboxylic acid of the formula

$$HOOC—R^1—COOH \qquad (III)$$

in which $R^1$ has the meaning indicated in formula I, the ratio of oxalkylated primary fatty amine to dicarboxylic acid being 0.8 : 1 to 1 : 0.8, which comprises reacting the resulting reaction product with ethylene oxide, propylene oxide or mixtures of these two alkylene oxides, in each case together with a carboxylic acid having 2 to 6 carbon atoms, a hydroxycarboxylic acid having 2 to 6 carbon atoms and 1 to 3 OH groups, with phosphoric acid or with hydrochloric acid.

4. The use of the quaternary oxalkylated polyesters as claimed in one or more of claims 1 and 2, as a conditioning active compound in cosmetic preparations for the care of the hair.

**Revendications**

1. Polyesters alcoxylés quaternaires de formule générale

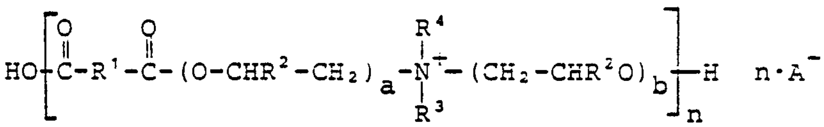

(I)

dans laquelle

$R^1$ est un radical alkylène de formule $—(CH_2)_m—$, où m est un nombre entier de 1 à 8, ce radical alkylène pouvant éventuellement porter 1 ou 2 groupes OH, ou bien un radical vinylène ou p-phénylène,

$R^2$ est H ou $CH_3$,

$R^3$ est un radical alkyle ou alcényle ayant de 8 à 23 atomes de carbone,

$R^4$ est un radical de formule $—(CH_2CHR^2O)_c—H$, où c est un nombre entier ou fractionnaire de 1 à 3, et $R^2$ a les significations ci-dessus,

$A^-$ est l'anion d'un acide carboxylique ayant de 2 à 6 atomes de carbone, d'un acide hydroxycarboxylique ayant de 2 à 6 atomes de carbone et 1 à 3 groupes OH, ou bien l'anion chlorure ou l'anion hydrogénophosphate,

a et b, identiques ou différents, sont chacun un nombre entier ou fractionnaire de 1 à 10, et

n est un nombre entier ou fractionnaire, qui peut prendre des valeurs de 5 à 60.

2. Polyesters alcoxylés quaternaires selon la revendication 1, caractérisés en ce que

$R^1$ est un radical alkylène de formule $—(CH_2)_m—$, où m peut prendre des valeurs de 4 à 8,

$R^2 = H$,

$R^3$ est un radical alkyle ayant de 16 à 23 atomes de carbone,

$R^4$ est un radical de formule $—(CH_2CH_2O)_c—H$, où c est un nombre entier ou fractionnaire de 1 à 3,

a et b, identiques ou différents, sont chacun un nombre entier ou fractionnaire de 2 à 4,

n est un nombre entier ou fractionnaire de 5 à 20, et

$A^-$ est l'anion de l'acide lactique, de l'acide tartrique ou de l'acide phosphorique.

3. Procédé pour la préparation de polyesters alcoxylés quaternaires de formule I selon la revendication 1, dans lequel on estérifie avec polycondensation une amine grasse primaire alcoxylée de formule

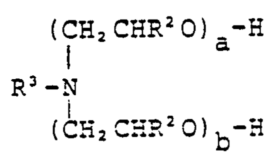

(II)

dans laquelle $R^3$, $R^2$, a et b ont les significations données dans la formule I, sur un acide dicarboxylique de formule

$$HOOC—R^1—COOH \qquad (III)$$

dans laquelle $R^1$ a les significations données dans la formule I, le rapport entre l'amine grasse primaire alcoxylée et l'acide dicarboxylique étant compris entre 0.8 : 1 et 1 : 0.8, caractérisé en ce qu'on fait réagir le produit de réaction obtenu sur de l'oxyde d'éthylène, de l'oxyde de propylène ou des mélanges de ces

deux oxydes d'alkylène, dans chaque cas en même temps qu'un acide carboxylique ayant de 2 à 6 atomes de carbone, un acide hydroxycarboxylique ayant de 2 à 6 atomes de carbone et 1 à 3 groupes OH, l'acide phosphorique ou l'acide chlorhydrique.

4. Utilisation des polyesters alcoxylés quaternaires selon une ou plusieurs des revendications 1 et 2 comme matière active pour le conditionnement de préparations de cosmétiques capillaires.